# EUROPEAN PATENT APPLICATION

(11) **EP 1 693 049 A1**
(43) Date of publication of application: **23.08.2006**
(21) Application number: 04820203.0
(22) Date of filing: 07.12.2004
(51) Int. Cl.: A61K 8/18

(54) **CAPSULE-CONTAINING ORAL COMPOSITION**

(30) Priority: 09.12.2003 JP 2003436434
(71) Applicant: SUNSTAR INC., Takatsuki-shi, Osaka 569-1195 (JP)
(72) Inventor: YASUDA, N., c/o SUNSTAR Inc., Takatsuki-shi, Osaka 569-1044 (JP); INOUE, A., c/o SUNSTAR Inc., Takatsuki-shi, Osaka 569-1044 (JP)
(74) Representative: TBK-Patent
(86) International application number: PCT/JP2004/018206
(87) International publication number: WO 2005/055967

(57) **Abstract**

The present invention provides an oral composition comprising capsule particles that are easily breakable to thereby readily release and distribute the capsule contents when used, while leaving substantially no feeling of capsule component residues or foreign matter in the mouth when rinsing. More specifically, the present invention relates to a capsule-containing oral composition comprising capsules with a capsule breaking point strength of 0.05 N or less when measured using a rheometer.

## Description

### TECHNICAL FIELD

The present invention relates to a capsule-containing oral composition comprising capsules whose contents include an oil-based ingredient. More specifically, the present invention relates to a capsule-containing oral composition comprising capsules which are breakable to thereby readily release and disperse the ingredient content when used for effective work in the oral cavity.

### BACKGROUND ART

Particles have conventionally been contained in dentifrice compositions to aid in the removal of dental plaque due to their abrasive properties and collapse effects (e.g. patent documents 1 to 3). However, these particles have a relatively high capsule breaking point strength and therefore remain in the mouth causing a sensation of foreign matter.

Many techniques in mixing capsules in oral compositions have also been reported.

For example, patent documents 4 to 8 disclose dentifrice compositions containing capsules enclosing perfumes, etc. These capsules are supposed to be easily breakable when the dentifrice compositions are used for brushing the teeth and the like, however, these particles are adapted to have an improved membrane strength by thickening and the like to prevent them from rupturing in the manufacturing process. For this reason, these particles are likely to remain in the oral cavity after use, leaving a sensation of foreign matter.

Patent documents 9 to 11 disclose dentifrice compositions whose capsule particles easily rupture, however, all these compositions leave capsule membranes in the mouth.

Patent document 12 discloses fine capsule particles with an average particle size of 1 to 50 µm comprising a N-vinylpyrrolidone-based polymer, however a problem is that such capsule particles are hard to rupture.

Further, patent document 13 discloses an oral composition containing large capsule particles with an average particle size exceeding 3 mm, however, it is difficult to rupture such capsule particles in the manners usually employed with oral compositions such as brushing the teeth, and the like.

No disclosure about oral compositions containing capsules that easily break and leave no capsule membrane residues when in use has been found in the above documents.
[Patent document 1]
Unexamined Japanese Patent Publication No. 1989-38016
[Patent document 2]
Unexamined Japanese Patent Publication No. 1992-368319
[Patent document 3]
Unexamined Japanese Patent Publication No. 2003-63939
[Patent document 4]
Unexamined Japanese Patent Publication No. 1974-453
[Patent document 5]
Examined Japanese Patent Publication No. 1988-48580
[Patent document 6]
Examined Japanese Patent Publication No. 1993-58404
[Patent document 7]
Examined Japanese Patent Publication No. 1975-25011
[Patent document 8]
Unexamined Japanese Patent Publication No. 1986-225115
[Patent document 9]
Unexamined Japanese Patent Publication No. 1996-169813
[Patent document 10]
Unexamined Japanese Patent Publication No. 1998-67625
[Patent document 11]
Unexamined Japanese Patent Publication No. 2000-302654
[Patent document 12]
Unexamined Japanese Patent Publication No. 2000-319151
[Patent document 13]
Unexamined Japanese Patent Publication No. 2002-20252

### DISCLOSURE OF THE INVENTION

### PROBLEMS TO BE SOLVED BY THE INVENTION

An object of the present invention is to provide an oral composition comprising easily rupturable capsule particles, which readily releases and distributes the capsule contents when in use, and leaves substantially no sensation of capsule component residues or foreign matter in the mouth when rinsing.

### MEANS FOR SOLVING THE PROBLEMS

The inventors have conducted extensive research to achieve the above object, and found that the oral composition comprising capsules with a capsule breaking point strength of 0.05 N or less attain the above objects. The inventors have conducted further study and accomplished the present invention.

Accordingly, the present invention provides the following capsule-containing oral compositions.
Item 1. A capsule-containing oral composition comprising capsules with a capsule breaking point strength of 0.05 N or less when measured using a rheometer.
Item 2. A capsule-containing oral composition according to Item 1, wherein the capsule contents include one or more oil-based ingredients, and each capsule membrane comprises one or more components selected from the group consisting of alginate, xanthan gum, agar, carrageenan, gellan gum, glucomannan, and locust bean gum.
Item 3. A capsule-containing oral composition according to Item 1 or 2, wherein each capsule membrane comprises one or more components selected from the group consisting of alginate, carrageenan and agar.
Item 4. A capsule-containing oral composition according to any one of Items 1 to 3, wherein the capsules have particle sizes ranging from 0.3 to 3.0 mm.
Item 5. A capsule-containing oral composition according to any one of Items 1 to 4, wherein the capsules are contained in an amount of 0.5 to 5.0 wt. % of the total weight of the composition.
Item 6. A capsule-containing oral composition according to any one of Items 1 to 5, wherein the composition is in the form of a toothpaste, liquid dentifrice, dental rinse, or mouthwash.

The present invention will be described below in details.

The capsule used in an oral composition of the invention has a capsule breaking point strength of not greater than 0.05 N, and preferably 0.001 to 0.03 N, when measured using a rheometer. A capsule within such a capsule breaking point strength range ruptures very easily, and leaves substantially no feeing of capsule component residues in the oral cavity.

Examples of capsule membrane components include alginate, xanthan gum, agar, carrageenan, gellan gum, glucomannan, locust bean gum, etc., with alginate, gellan gum, carrageenan, agar, locust bean gum, etc. being preferable, and alginate, carrageenan and agar being particularly preferable. The capsule membrane may be one or more components selected from the group consisting of these materials.

The capsule breaking point strength of the capsule used in the invention means the capsule breaking point strength of the capsule contained in the oral composition measured using a rheometer. More specifically, capsules are separated from the oral composition at least three hours after they are admixed therein, and a load is applied to each capsule until it ruptures. Capsule breaking point strength is expressed by the force (N) at which the capsule ruptures.

The average particle size of the capsules used in the invention is about 0.3 to about 3.0 mm, preferably 0.4 to 2.8 mm, and particularly preferably 0.5 to 2.5 mm. Capsules do not easily break when the average particle size is below 0.3 mm, whereas they cause a strong feeling of foreign matter in the mouth in addition to being difficult to break when the average particle size is greater than 3 mm.

The capsule used in the invention has an oil-based ingredient content. Oil-based ingredients included are not limited so long as they can be used for oral compositions, and may suitably be selected in accordance with purpose from, for example, menthol, clove oil, carvone, limonene, citronellol, cineole, linalool, anethole, and like perfume ingredients; vitamin E, vitamin D, vitamin A, retinol, β-carotene, vitamin F, and like oil-soluble vitamins; triclosan, isopropylmethylphenol, coenzymes Q, glycyrrhetic acid, glycyrrhizic acid, and like fat-soluble active ingredients; thymol, spearmint oil, peppermint oil, lemon oil, orange oil, eucalyptus oil, rose oil, and like oil-soluble plant extracts; olive oil, silicone oil, and like functional oils; and Red No.215, Yellow No.204, Green No.202, and like oil-soluble solvent dyes.

The oil-based ingredient-including capsules are produced, for example, by a method wherein an aqueous mixture (emulsion, solution, etc.) containing capsule membrane component(s), an oil-based ingredient(s), etc. is added dropwise to a solution containing a curing agent such as a calcium salt for causing granulation (falling-drop method). More specifically, an example is a method by which an emulsion containing sodium alginate, an oil-based ingredient, and water is added dropwise to an aqueous calcium chloride solution.

Such capsules having an oil-based ingredient content are contained in 0.5 to 5.0 wt %, preferably 1.0 to 3.0 wt %, and more preferably 1.5 to 3.0 wt. % of the total weight of the capsule-containing oral composition of the invention. When not enough capsules are contained, a sufficient amount of particles do not rupture with a single brushing of the teeth, failing to impart the feeling of the particles' effects. In contrast, an excessive amount of the particles impairs appearance of the composition.

The capsule-containing oral composition of the invention is produced in the same manner as employed for producing known oral compositions. More specifically, for example, capsules may be incorporated into the oral composition so as to be within a proportional range mentioned above, and uniformly mixed. Needless to say, the composition should be prepared in such a manner that capsules are prevented from rupturing in the production process.

In the capsule-containing oral composition of the invention, one or more surfactants selected from the group consisting of anionic surfactants, nonionic surfactants, and amphoteric surfactants may be contained in the composition but not within the capsules. In particular, one or more anionic surfactants are preferably selected to better attain suitable capsule breaking point strength. In addition to such anionic surfactants, one or more surfactants selected from the group consisting of nonionic surfactants and amphoteric surfactants may be contained.

Anionic surfactants used in the invention are not limited, and examples include sodium lauryl sulfate, sodium myristyl sulfate, N-lauroyl sarcosinate, lauroyl methyl taurine, lauryl sulfosuccinates, etc., with sodium lauryl sulfate being particularly preferable.

Nonionic surfactants used in the invention are not limited, and examples include cane sugar fatty acid esters, myristic acid diethanolamide, polyoxyethylene hardened castor oils, polyoxyethylene polyoxypropylene glycols, alkyl glucosides, polyglycerol fatty acid esters, etc., with polyoxyethylene hardened castor oils being particularly preferable.

Usable amphoteric surfactants are, for example, N-alkyldiamino ethyl glycine, betaine surfactants, etc.

The proportions of these anionic, nonionic and amphoteric surfactants are not limited so long as the objects of the invention are attained, and may be, based on the total weight of the capsule-containing oral composition of the invention, 0.05 to 3.0 wt. %, and preferably 0.5 to 2.0 wt. % of anionic surfactants, 0.1 to 3.0 wt. %, and preferably 0.5 to 2.0 wt. % of nonionic surfactants, and 0.1 to 3.0 wt. %, and preferably 0.5 to 2.5 wt. % of amphoteric surfactants.

The pH of the capsule-containing oral composition of the invention is from 5.5 to 8.0, and preferably from 6.0 to 7.5. A pH of 5.5 or less may cause melting of dentin, whereas a pH of 8.0 or higher causes problems with capsule stability.

Capsule-containing oral compositions of the invention can be made in the form of toothpastes, liquid dentifrices, dental rinses, mouthwashes, and the like. Among these, toothpastes, liquid dentifrices and mouthwashes are preferable.

The capsule-containing oral composition of the invention may suitably contain known components such as abrasives, humectants, dispersants, surfactants, active ingredients, flavoring agents, colorants, etc., in accordance with the form desired.

Specific examples of abrasives include precipitated silica, zircon silicate, aluminosilicate, calcium phosphate, calcium carbonate, aluminium hydroxide, aluminium oxide, etc. Such abrasives are usually about 1 to about 40 wt. % of the total amount of the composition.

Examples of humectants include ethanol, glycerol, sorbitol, ethylene glycol, propylene glycol, 1,3-butylene glycol, polyethylene glycol, polypropylene glycol, xylitol, maltitol, lactitol, etc. Such humectants are usually about 2 to about 70 wt. % of the total amount of the composition.

Examples of dispersants include cellulose derivatives such as sodium alginate, carrageenan, sodium carboxymethyl cellulose, methyl cellulose, hydroxyethyl cellulose, etc.; gums such as tragacanth gum, karaya gum, gum arabic, xanthan gum, gellan gum, etc.; synthetic water-soluble polymers such as sodium polyacrylate, polyvinyl alcohol, carboxy vinyl polymers, polyvinylpyrrolidone, etc.; inorganic dispersants such as silica gel, alumino-silica gel, VEEGUM, Laponite, etc. Such dispersants are usually contained about 0.005 to about 1 wt. % of the total amount of the composition.

Examples of active ingredients include fluorine compounds such as sodium fluoride, sodium monofluorophosphate, stannous fluoride, etc.; enzymes such as dextranase, mutanase, protease, lysozyme, etc.; anti-inflammatory drugs such as tranexamic acid, ε-aminocaproic acid, allantoin-chlorohydroxy aluminum, dihydrocholestanol, glycyrrhizinates, bisabolol, etc.; antibacterial drugs such as isopropylmethylphenol, chlorohexidine salts, triclosan, cetylpyridinium chloride, benzalkonium chloride, benzethonium chloride, etc.; plant extracts such as cloves, rosemary, thyme, scutellaria root, etc.; antiplaque agents such as water-soluble inorganic phosphate compounds, etc.; breath freshening agents such as chlorophyll, copper gluconate, etc.; and other ingredients such as glycerophosphates, sodium chloride, etc.

Examples of flavoring agents include saccharin sodium, stevioside, glycyrrhizin, aspartylphenylalanine methyl ester, menthol, anethole, carvone, eugenol, limonene, peppermint, spearmint, winter green, etc.

Examples of colorants include Blue No. 204, Blue No. 203, etc.

### EFFECT OF THE INVENTION

The capsule-containing oral composition of the invention comprises capsules that are easily rupturable when used, readily releases and disperses the enclosed oil-based ingredient(s), and leaves no capsule component residues as foreign matter in the mouth while rinsing, and therefore has excellent cleaning properties, and imparts an extremely comfortable sense of use and refreshing sensation.

### BEST MODE FOR CARRYING OUT THE INVENTION

The present invention will be described in further details with reference to Examples and Test Examples below. However, the invention is not limited to these examples. Unless otherwise stated, "%" means "wt. %."

The capsule breaking point strength (N) of the capsule of the invention is a value measured using a rheometer. More specifically, the capsule breaking point strength is expressed by the force (N) at which the capsule ruptures when a load is applied thereto. Rupture of a capsule means when the contents are confirmed to have been spilled from the capsule.

### [Capsule Preparation]

### Capsule 1

A mixture of sodium alginate, propylene glycol alginate, perfume and water was emulsified in a blender, and the emulsion was added dropwise to an aqueous solution of calcium chloride to obtain Capsule 1 (particle size: 1.0 mm, contents: perfume).

### Capsule 2

A mixture of carrageenan, perfume and water was heated with stirring until homogenized, and the mixture was added dropwise to an aqueous calcium lactate solution to obtain Capsule 2 (particle size: 0.5 mm, contents: perfume).

### Capsule 3

A mixture of gelatin, gum arabic and water was heated for dissolution, and perfume was added thereto. The mixture was emulsified in a blender, and the emulsion was added dropwise to a calcium chloride solution to obtain Capsule 3 (particle size: 1.0 mm, contents: perfume).

### Capsule 4

A mixture of sodium alginate, agar, triclosan and water was emulsified in a blender, and the emulsion was added dropwise to an aqueous solution of calcium chloride to obtain Capsule 4 (particule size: 3.0 mm, contents: triclosan).

### Capsule 5

A mixture of sodium alginate, gellan gum, clove oil and water was heated with stirring until homogenized. The mixture was added dropwise to an aqueous calcium lactate solution to obtain Capsule 5 (particle size: 2.0 mm, contents: clove oil).

Subsequently, mixtures were prepared using the ingredients in the proportions as shown in Table 1 to obtain capsule-containing toothpastes. The obtained capsusle-containing toothpastes were evaluated in terms of capsule breaking point strength, capsule content releasability and capsule residue feeling in the oral cavity after use. Below is each evaluation method, and the evaluation results are shown in Table 1.

### [Evaluation Methods]

### 1. Capsule breaking point strength (N) of capsule in toothpaste

Capsules were mixed into a dentifrice composition and kept for at least 3 hours at room temperature (1 to 30 °C), and subsequently separated from the composition. A removed capsule was washed in water to use as a sample for measurement. The capsule breaking point strength was measured using a rheometer (Sun Scientific Co., Ltd., Rheometer CR-200D). Capsule breaking point strength in the present invention is expressed by the force at which the capsule ruptures.

### 2. Releasability of capsule contents

Ten panelists took 1 g of each of the toothpastes prepared in Examples 1 and 2 and Comparative Example 1 on a toothbrush and brushed their teeth. Releasability of capsule contents by the panelists was evaluated based on the following criteria, and indicated by point average.

### < Evaluation Criteria >

5: Particles ruptured, with clearly sensed change in taste
4: Sensed change in taste
3: Faintly sensed change in taste
2: Sensed no change in taste
1: Particles not ruptured

### 3. Capsule Residue Feeling

Ten panelists took 1 g of each of the toothpaste prepared in Examples 1 an 2 and Comparative Example 1 on a toothbrush and brushed their teeth. After discharging the toothpaste, capsule residue feeling in the oral cavity was evaluated based on the following criteria, and the results were indicated by point averages.

### < Evaluation Criteria >

5: Sensed no residues at all
4: Sensed substantially no residues
3: Very faintly sensed residues
2: Faintly sensed residues
1: Sensed residues

**Table 1**

| Ingredient content (%) | Ex. 1 | Ex. 2 | Com. Ex. 1 |
|---|---|---|---|
| Silicic anhydride | 19 | 19 | 19 |
| Sodium carboxymethyl cellulose | 0.8 | 0.8 | 0.8 |
| Magnesium sulfate | 2 | 2 | 2 |
| Sodium monofluorophosphate | 0.7 | 0.7 | 0.7 |
| Sorbitol | 59 | 59 | 59 |
| Polyethylene glycol | 4 | 4 | 4 |
| Sodium saccharin | 0.2 | 0.2 | 0.2 |
| Sodium phosphate, anhydrous | 0.15 | 0.15 | 0.15 |
| Sodium lauryl sulfate | 2 | 2 | 2 |
| Polyoxyethylene hardened castor oil | 1 | 1 | 1 |
| Perfume | 0.5 | 0.5 | 0.5 |
| Capsule 1 | 2.5 | - | - |
| Capsule 2 | - | 1.0 | - |
| Capsule 3 | - | - | 2.5 |
| Water | Remainder | Remainder | Remainder |
| Total | 100 | 100 | 100 |
| Capsule breaking point strength (N) in composition | 0.01 | 0.02 | 1.5 |
| Perceived released perfume | 4.8 | 4.2 | 1.3 |
| Perceived capsule residues | 4.6 | 4.0 | 2.1 |

The following Examples 3 to 6 show specific forms of the capsule-containing oral composition of the invention. All the forms in Examples 3 to 6 were produced by known methods. In all the forms, the capsule contents were readily released and no sensation of capsule residues was perceived in the mouth when used.

### Example 3 Toothpaste

**Table 2**

| Ingredient | Content (%) |
|---|---|
| Silicic anhydride | 10.0 |
| Xanthan gum | 0.7 |
| Magnesium sulfate | 2.0 |
| Sodium monofluorophosphate | 0.7 |
| Sorbitol | 59.0 |
| Polyethylene glycol | 4.0 |
| Sodium saccharin | 0.2 |
| Sodium lauryl sulfate | 1.0 |
| Perfume | 0.5 |
| Capsule 4 | 1.0 |
| Water | Remainder |
| Total | 100.0 |

### Example 4 Toothpaste

**Table 3**

| Ingredient | Content (%) |
|---|---|
| Silicic anhydride | 15.0 |
| Sodium carboxymethyl cellulose | 1.0 |
| Sorbitol | 40.0 |
| Concentrated glycerol | 7.0 |
| Propylene glycol | 1.0 |
| Sodium saccharin | 0.1 |
| N-lauroyl sarcocinate | 0.5 |
| Alkyl glycoside(s) | 2.0 |
| Perfume | 0.7 |
| Capsule 5 | 3.0 |
| Water | Remainder |
| Total | 100.0 |

### Example 5 Liquid toothpaste

**Table 4**

| Ingredient | Content (%) |
|---|---|
| Sodium monofluorophosphate | 0.7 |
| Sorbitol | 54.0 |
| Concentrated glycerol | 2.0 |
| Ethanol | 5.0 |
| Magnesium sulfate | 2.0 |
| Sodium saccharin | 0.2 |
| Carrageenan | 0.5 |
| Sodium lauryl sulfate | 0.5 |
| Polyglycerol fatty acid ester(s) | 2.0 |
| Perfume | 0.9 |
| Capsule 1 | 2.0 |
| Water | Remainder |
| Total | 100.0 |

### Example 6 Mouthwash

**Table 5**

| Ingredient | Content (%) |
|---|---|
| Triclosan | 0.02 |
| Concentrated glycerol | 15.0 |
| Ethanol | 7.0 |
| Magnesium sulfate | 2.0 |
| Sodium saccharin | 0.2 |
| Sodium lauryl sulfosuccinate | 0.5 |
| Polyoxyethylene hardened castor oil | 2.0 |
| Perfume | 0.5 |
| Capsule 2 | 1.5 |
| Water | Remainder |
| Total | 100.0 |

## Claims

1. A capsule-containing oral composition comprising capsules with a capsule breaking point strength of 0.05 N or less when measured using a rheometer.

2. A capsule-containing oral composition according to Claim 1, wherein the capsule contents include one or more oil-based ingredients, and each capsule membrane comprises one or more components selected from the group consisting of alginate, xanthan gum, agar, carrageenan, gellan gum, glucomannan, and locust bean gum.

3. A capsule-containing oral composition according to Claim 2, wherein each capsule membrane comprises one or more components selected from the group consisting of alginate, carrageenan and agar.

4. A capsule-containing oral composition according to Claim 1, wherein the capsules have particle sizes ranging from 0.3 to 3.0 mm.

5. A capsule-containing oral composition according to Claim 1, wherein the capsules are contained in an amount of 0.5 to 5.0 wt. % of the total weight of the composition.

6. A capsule-containing oral composition according to any one of Claims 1 to 5, wherein the composition is in the form of a toothpaste, liquid dentifrice, dental rinse, or mouthwash.
